# EUROPEAN PATENT APPLICATION

(11) **EP 3 594 682 A1**
(43) Date of publication of application: **15.01.2020**
(21) Application number: 18197561.6
(22) Date of filing: 28.09.2018
(51) Int. Cl.: G01N 33/44, G01N 21/88

(54) **LEATHER INSPECTION EQUIPMENT**

(30) Priority: 12.07.2018 TW 107124163; 18.09.2018 TW 107132718
(71) Applicant: Aibi Dynamics Co., Ltd., 407 Taichung City (TW)
(72) Inventor: CHANG, Yu-Pin, 407 Taichung City (TW)
(74) Representative: Becker Kurig Straus

(57) **Abstract**

A leather inspection equipment includes a leather inspection platform (12) for the placement of a leather raw material (10) to be inspected, and a leather data collection device (14) installed in the leather inspection platform (12) to detect the leather raw material (10), thereby obtaining a digital leather data for calculation by an artificial intelligence module (20) to judge the surface state of the leather raw material (10). Thus, you can greatly reduce the leather inspection time and increase production efficiency, and at the same time realize the automatic production process of leather products.

## Description

### BACKGROUND OF THE INVENTION

### 1. Field of the Invention

The present invention relates to leather technology and more particularly, to a leather inspection equipment.

### 2. Description of the Related Art

Leather can be used in a wide range of consumer goods, such as clothing, purses, luggage or decorative accessories, etc., which are everyday items that are often used. Because natural leather (also known as genuine leather) has good touch and high durability, high-priced and high-value products often use natural leather as the main material.

Natural leather is susceptible to damage from raw materials or manufacturing processes, such as damage and defects on the leather surface or internal tissues due to animal injury, mildew, pests, ruptures, or transport bumping. In order to allow the leather products to be inspected beforehand, most of the time, when the leather is still in the raw material state, the leather is inspected in detail by visual inspection or manual inspection, and then the leather surface is marked to indicate the defective areas. The inspected and marked leathers are then properly cut and sorted into the available leather parts for subsequent production according to the required characteristics of the finished product.

However, the above-mentioned inspection method using human visual inspection or hand inspection is not only time consuming, it needs to employ an inspector with sufficient experience to judge the defect, and the training and cultivation process of the inspection personnel is long and difficult. Because the judgment method relies on more subjective visual or sensory inspection, it is difficult to establish more consistent and universal quality standards due to factors such as personal emotion, environment or time and space.

### SUMMARY OF THE INVENTION

The present invention has been accomplished under the circumstances in view. It is the main object of the present invention to provide a leather inspection equipment, which can greatly reduce the leather inspection time and increase production efficiency, and at the same time realize the automatic production process of leather products.

To achieve this and other objects of the present invention, a leather inspection equipment comprises a leather inspection platform and a leather data collection device. The leather inspection platform is adapted for flat placement of a leather raw material to be inspected. The leather data collection device is installed in the leather inspection platform to detect the leather raw material, thereby obtaining a relative digital leather data.

Preferably, the leather data collection device comprises at least one sensor component for sensing the leather raw material.

Preferably, the said at least one sensor component detects the leather raw material by reflection or transmission.

Preferably, the leather data collection device comprises a plurality of sensor components arranged in an array and evenly distributed over the leather raw material on the leather inspection platform to respectively obtain a digital local leather data of one respective local area of the leather raw material.

Preferably, the leather inspection equipment further comprises an image processing module for integrating the digital local leather data obtained by each sensor component into a complete digital leather data.

Preferably, the leather inspection further comprises an artificial intelligence module for calculating the digital leather data to judge the surface state of the leather raw material.

Preferably, the leather inspection platform comprises a display device for displaying the digital leather data captured by the leather data collection device.

Preferably, the leather inspection platform comprises a control unit operable to turn on/off said leather data collection device.

Other advantages and features of the present invention will be fully understood by reference to the following specification in conjunction with the accompanying drawings, in which like reference signs denote like components of structure.

### BRIEF DESCRIPTION OF THE DRAWINGS

FIG. 1 is an oblique top elevational view of a leather inspection equipment in accordance with the present invention.
FIG. 2 is a schematic drawing of the present invention, illustrating the configuration of the leather inspection platform.
FIG. 3 is similar to FIG. 2, illustrating a leather raw material placed on the leather inspection platform.
FIG. 4 is a system block diagram of the present invention.
FIG. 5 is a schematic drawing of the present invention, illustrating the configuration of an alternate form of the leather data collection device.

### DETAILED DESCRIPTION OF THE INVENTION

Prior to viewing the following specification in conjunction with the accompanying drawings, it is to be understood that the leather inspection equipment of the present invention can be widely used to inspect various types or surface treatments of natural leather or synthetic leather. Those skilled in the art should be able to understand that the composition components and explanatory language in the present preferred embodiment are all superordinated descriptions that do not limit the specific devices or the technical field. And for the quantitative term "a" is meant to include one and more than a plurality of components.

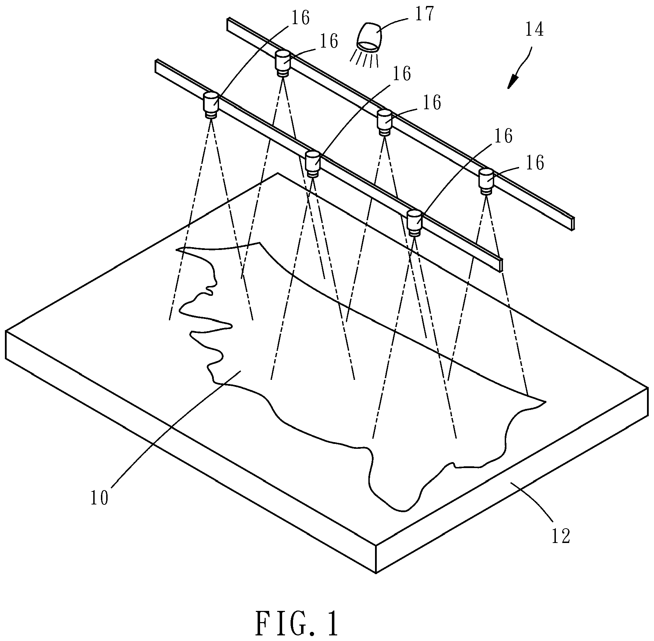

Referring to FIG. 1, a leather inspection equipment mainly comprises a leather inspection platform **12** and a leather data collection device **14.** The leather inspection platform **12** is adapted for flat placement of a leather raw material **10.**

The leather data collection device **14** is disposed near the leather inspection platform **12.** In the present preferred embodiment, the leather data collection device **14** is disposed above the leather inspection platform **12** to obtain digital leather data of the leather raw material **10.**

In the present preferred embodiment, the leather raw material **10** is a natural leather, however, the leather inspection equipment is also applicable to other kinds of leathers.

In the present preferred embodiment, the leather data collecting device **14** comprises at least one optical sensor device. The leather data collecting device **14** photographs the surface of the leather raw material **10** to obtain a digital image of the leather surface so as to further form digital leather data for judging the edge and surface defect state of the leather raw material **10.**

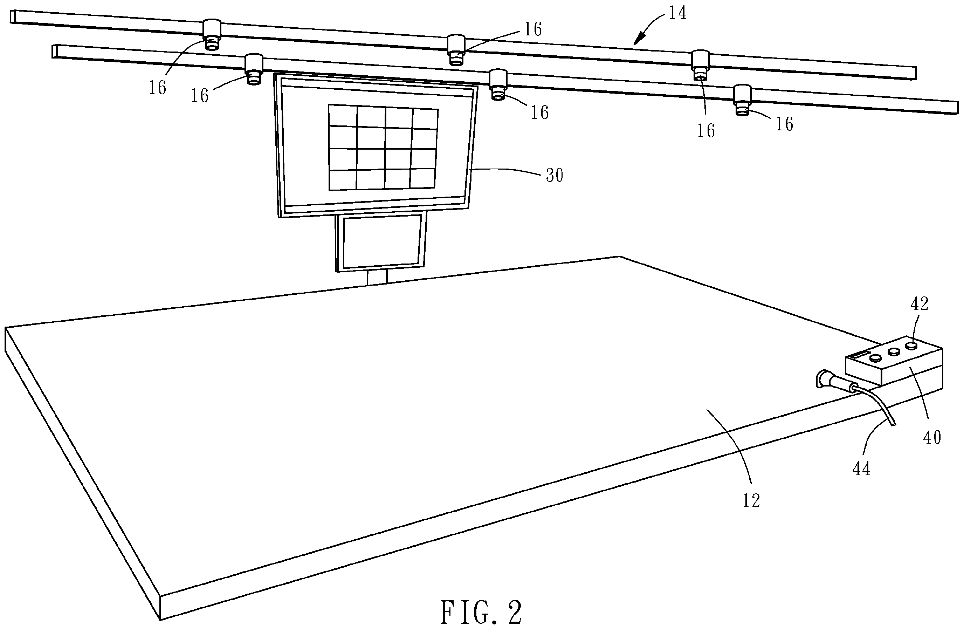

As shown in FIGS. 1-3, the leather data collecting device **14** comprises a plurality of sensor components **16** that are arranged in an array and evenly distributed over the leather raw material **10.** Each of the sensor components **16** obtains the digital local leather data of one respective local area of the leather raw material **10.** The sensor components **16** can be digital cameras or the like. The leather data collecting device **14** further comprises a light source **17** adapted to emit light onto the leather raw material **10** for enabling the sensor components **16** to obtain complete digital leather data. The light source **17** can be a point light source or an array light source. In order to cooperate with the material types, different material treatments or different leather raw material surface textures, the light source **17** can project different illumination characteristics according to the material characteristics of different leather raw materials **10,** that is, the intensity, illuminance, or brightness of the light source can be adjusted with the leather raw material **10** to be inspected.

The leather inspection platform **12** is equipped with a display device **30** and a control unit **40.** The display device **30** is adapted for displaying the leather image captured by the leather data collection device **14.** The control unit **40** comprises an operating device **42** and a scanner **44.** The operating device **42** is used to turn on/off the leather data collection device **14.** The scanner **44** is used to produce a process record for the leather raw material **10.**

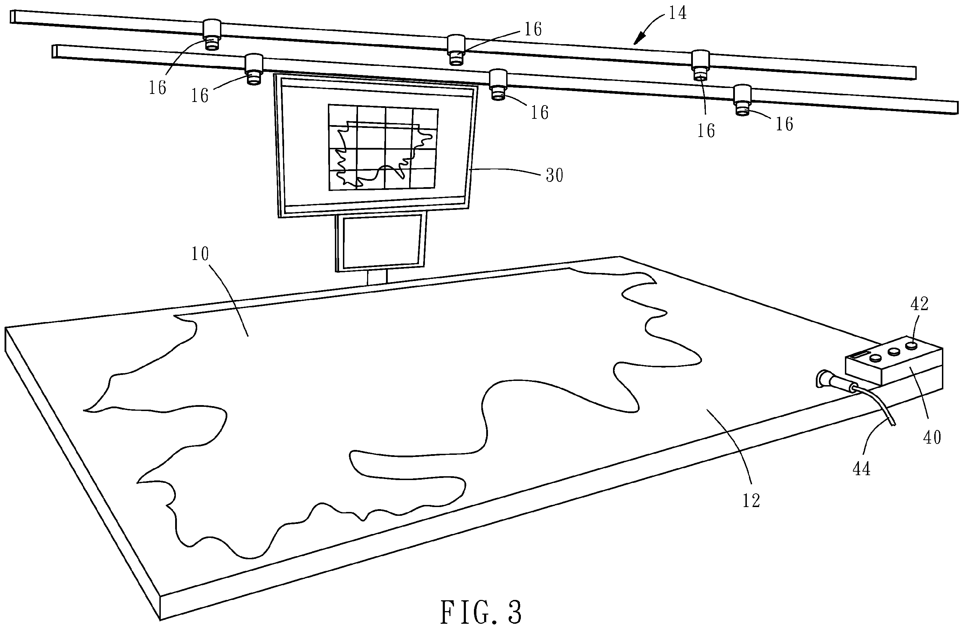

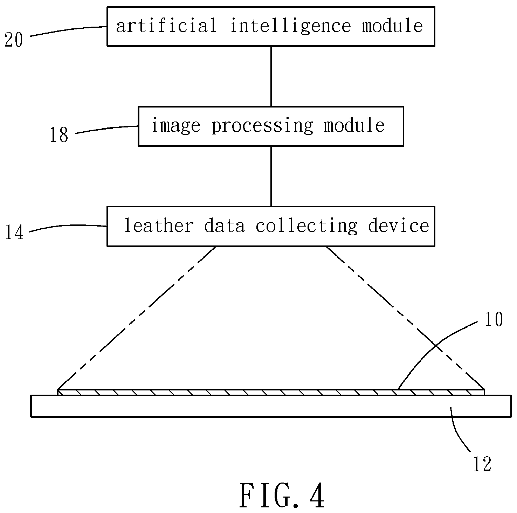

As illustrated in FIG. 3 and FIG. 4, after the leather raw material **10** was placed flat on the leather inspection platform **12** and the leather data collection device **14** above the leather inspection platform **12** captured the local digital leather data of each of various areas of the leather raw material **10,** all the local digital leather data captured by the leather data collection device **14** are then processed through an image processing module **18** that splices and integrates all the local digital leather data into a complete digital leather data.

The complete digital leather data can also be used to calculate and judge the surface state of the leather raw material **10** through an artificial intelligence module **20.** The artificial intelligence module **20** of the present preferred embodiment uses a deep learning model as an example. After the digital leather data is judged by the artificial intelligence module **20,** the surface state of the leather raw material **10** can then be used to match the subsequent leather product production process.

Through the above-mentioned constituent members of the present invention, the leather data collection device utilizes the sensor components to comprehensively capture the digital image of the surface of the leather raw material, thereby digitizing the leather characteristics and facilitating the subsequent use of the artificial intelligence module with the deep learning model to judge the characteristics of the leather and to achieve the purpose of greatly reducing the leather inspection time. Through the same inspection equipment, you can quickly complete the inspection without considering the inspection environment, time or manpower, and establish a consistent and universal leather quality inspection standard.

It is worth mentioning that the above-mentioned leather data collection device can also obtain the internal organization or material state of the leather raw material by using a device to produce a collapse effect on leather raw material by means of transmission or mechanical force. For example, use an X-ray device to irradiate X-rays to the leather raw material, and then analyze the X-ray signal thus obtained to find out the characteristics of the inner organization of the leather raw material.

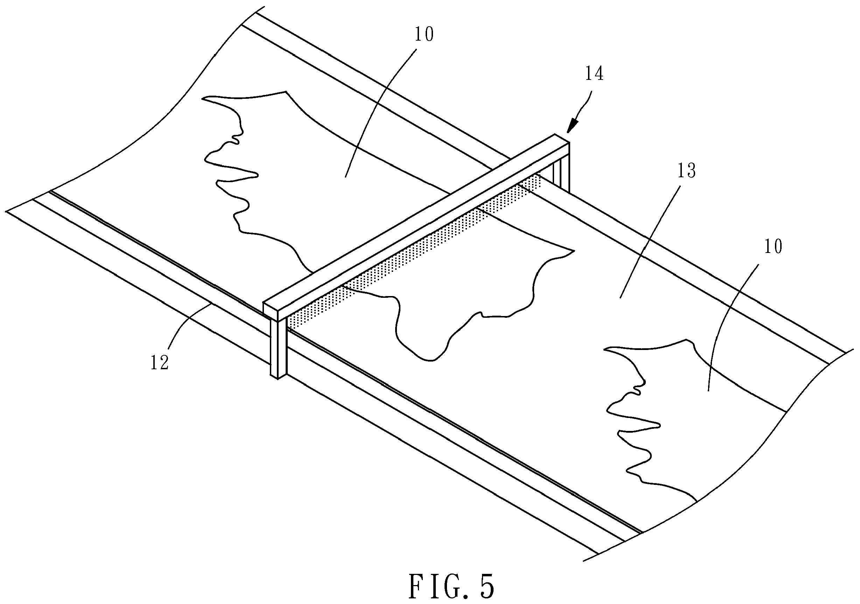

As shown in FIG. 5, the leather data collecting device **14** can scan the leather surface of the leather raw material **10** to obtain the desired leather data when the leather raw material **10** is moving along with a conveyor belt **13** over the leather inspection platform **12,** thereby improving the overall inspection and production efficiency.

## Claims

1. A leather inspection equipment, which is **characterized in that** said leather inspection equipment comprises:
a leather inspection platform (12) for the placement of a leather raw material (10) to be inspected; and
a leather data collection device (14) installed in said leather inspection platform (12) to detect said leather raw material (10), thereby obtaining a relative digital leather data.

2. The leather inspection equipment as claimed in claim 1, wherein said leather data collection device (14) comprises at least one sensor component (16) for sensing said leather raw material (10).

3. The leather inspection equipment as claimed in claim 2, wherein said at least one sensor component (16) detects said leather raw material (10) by reflection or transmission.

4. The leather inspection equipment as claimed in claim 1, wherein said leather data collection device (14) comprises a plurality of sensor components (16) arranged in an array and evenly distributed over said leather raw material (10) on said leather inspection platform (12) to respectively obtain a digital local leather data of one respective local area of said leather raw material (10).

5. The leather inspection equipment as claimed in claim 4, further comprising an image processing module (18) for integrating the digital local leather data obtained by each said sensor component (16) into the complete said digital leather data.

6. The leather inspection equipment as claimed in claim 1, wherein said leather data collection device (14) comprises a light source (17) adapted for projecting light onto said leather raw material (10), the lighting characteristics of said light source (17) being adjustable according to the material characteristics of said leather raw material (10).

7. The leather inspection equipment as claimed in claim 1, further comprising an artificial intelligence module (20) for calculating said digital leather data to judge the surface state of said leather raw material (10).

8. The leather inspection equipment as claimed in claim 1, wherein said leather inspection platform (12) comprises a display device (30) for displaying the digital leather data captured by said leather data collection device (14).

9. The leather inspection equipment as claimed in claim 1, wherein said leather inspection platform (12) comprises a control unit (40) operable to turn on/off said leather data collection device (14).
